# FASCICULE DE BREVET EUROPEEN

(11) **EP 2 252 892 B1**
(45) Date de publication et mention de la délivrance du brevet: **29.10.2014**
(21) Numéro de dépôt: 09729050.6
(22) Date de dépôt: 12.03.2009
(51) Int. Cl.: G01N 33/569, C12Q 1/04

(54) **PROCEDE DE DETECTION EN TEMPS REEL DE MICROORGANISMES DANS UN MILIEU DE CULTURE LIQUIDE PAR AGGLUTINATION**
VERFAHREN ZUM ECHTZEITNACHWEIS VON MIKROORGANISMEN IN EINEM FLÜSSIGEN KULTURMEDIUM DURCH AGGLUTINIERUNG
METHOD FOR THE REAL-TIME DETECTION OF MICROORGANISMS IN A LIQUID CULTURE MEDIUM BY AGGLUTINATION

(30) Priorité: 14.03.2008 FR 0851655
(43) Date de publication de la demande: 24.11.2010
(73) Titulaire: Biomérieux, 69280 Marcy L'etoile (FR)
(72) Inventeur: COLIN, Bruno, F-69280 Marcy l'Etoile (FR); MOSTICONE, David, F-69280 Sainte Consorce (FR); RAYMOND, Jean-Claude, F-69690 Bessenay (FR); SOFIA, Thierry, F-69290 Saint-Genis-les-Ollières (FR); VIMONT, Antoine, 69005 Lyon (FR)
(74) Mandataire: Sprugnoli, Claude Louis Victor
(86) Numéro de dépôt international: PCT/FR2009/050415
(87) Numéro de publication internationale: WO 2009/122069

(56) Documents cités:
- WO-A-2005/068647
- US-A- 4 659 658
- US-A- 5 217 715
- US-A- 5 496 706
- US-A1- 2005 118 660
- US-A1- 2006 246 535

## Description

Le domaine de l'invention est celui du contrôle microbiologique clinique ou industriel. Plus particulièrement, il s'agit d'une méthode permettant d'identifier un ou plusieurs microorganismes par une réaction d'agglutination effectuée simultanément à l'enrichissement de l'échantillon en microorganismes.

L'analyse microbiologique nécessite des techniques précises et dont le temps d'obtention du résultat doit être le plus court possible.

Dans le domaine médical, il est nécessaire de prévoir et diagnostiquer le risque infectieux : plus le diagnostic est rapide et précis, plus la prise en charge des malades est efficace et le risque de transmission minimisé. L'approche est similaire pour la santé animale.

Dans le domaine agroalimentaire, le cahier des charges est identique. Il distingue cependant les microorganismes pathogènes et leurs toxines dont la recherche s'applique aux produits commercialisés, les microorganismes non pathogènes, utilisés comme indicateurs-qualité du processus de production, des produits bruts aux produits finis, tout au long de la chaîne, et les bactéries d'intérêt technologique telles les ferments. La détection rapide et précise de contaminations présumées permet de les contrôler et d'engager ainsi des actions correctives.

Techniquement, l'analyse microbiologique peut mettre en oeuvre une ou plusieurs phases de pré-enrichissement/enrichissement, une ou plusieurs phases de détection, une ou plusieurs phases d'énumération des microorganismes. Pour des applications particulières telles le contrôle microbiologique agroalimentaire, une phase de confirmation peut également être requise, afin de répondre aux normes en vigueur dans ce domaine.

La phase de pré-enrichissement/enrichissement fait appel à des milieux de culture, sélectifs ou non, bien connus de l'homme du métier. Des milieux de culture prêts à l'emploi, souvent sous forme liquide, basés sur des formules de milieux traditionnels sont disponibles commercialement.

La phase de détection se base sur la mise en évidence des caractères métaboliques des microorganismes recherchés. On utilise classiquement des substrats enzymatiques spécifiques. Ces substrats enzymatiques sont généralement composés de deux parties, une première partie spécifique de l'activité enzymatique à révéler, appelée également partie cible, et une seconde partie faisant office de marqueur, appelée partie marqueur, généralement constituée par un chromophore ou un fluorophore. Par le choix de ces substrats, selon qu'il y a réaction ou non, il est possible de caractériser la nature d'un microorganisme ou de discriminer différents groupes de microorganismes. Ainsi, l'apparition ou la disparition d'une coloration ou d'une fluorescence sera la signature d'un genre ou d'un type de microorganismes. A cet égard, l'utilisation de milieux chromogènes permet la détection et l'identification simultanées des germes recherchés. Elle simplifie le processus et diminue sensiblement le délai d'obtention du résultat. On citera à titre d'exemple concret les milieux ChromID® de la demanderesse. Ces milieux chromogènes sont basés sur la détection de caractères métaboliques spécifiques des germes recherchés comme par exemple l'activité enzymatique beta-glucuronidase pour *Escherichia coli.* Cependant, certains microorganismes, ou certains sous-types comme par exemple *Escherichia coli* O157 :H7, ne présentent pas d'activité enzymatique spécifique et ne peuvent donc pas être détectés à l'aide d'un milieu de culture chromogène.

La phase de confirmation, quant à elle, est plus particulièrement attachée à l'analyse microbiologique dans le domaine agroalimentaire. En effet, lorsque le résultat des méthodes développées précédemment est positif, il est nécessaire de confirmer la présence du pathogène recherché. Ceci impose un test complémentaire et l'utilisation d'un principe de détection différent de celui utilisé lors de la première analyse. Les techniques de biologie moléculaire, basées sur les caractères génomiques des microorganismes recherchés, constituent l'un des moyens utilisés pour confirmer l'identification. On citera à titre d'exemple les techniques d'amplification classiques telles la PCR (Polymerase Chain Reaction) et la NASBA (Nucleic Acid Sequence Based Amplification), qui peuvent être couplées à des techniques de détection en temps réel connues de l'homme du métier.

Les immuno-essais constituent une autre des technologies utilisées pour le test de confirmation. Ils font appel aux caractéristiques immunogènes des microorganismes recherchés. De façon non exhaustive, on peut citer les techniques ELISA (Enzyme Linked ImmunoSorbent Assay), par compétition ou de type sandwich, ou les techniques d'immunoagglutination, détectant des épitopes des microorganismes recherchés. Ces dernières font appel à des supports solides fonctionnalisés, tels des billes (par exemple des particules de latex) recouvertes d'anticorps monoclonaux ou polyclonaux, lesdits supports fonctionnalisés étant mis en contact avec un échantillon biologique, comme l'indiquent par exemple les brevets européens délivrés EP 0 701 624 et EP 1 199 567. Alternativement, comme décrit dans le brevet US-4,659,658, les particules solides peuvent être recouvertes de lectines se liant spécifiquement à des sucres situés à la surface d'un microorganisme donné US5217715 décrit une méthode consistant à fixer un récepteur hydrocarboné N-acétylgalactosamine-beta-1-4-galactose-beta-1-4-glucose sur un support insoluble pouvant être utilisé pour la détection de bactéries dans un test d'agglutination. Dans tous les cas, l'apparition de l'agglutination permet d'identifier de façon certaine le microorganisme recherché.

L'identification complète et précise d'un microorganisme dans un échantillon nécessite donc l'enchaînement de plusieurs étapes : enrichissement, détection confirmation. La standardisation des tests utilisés en routine a permis l'automatisation des méthodes de détection qui restent cependant longues à mettre en oeuvre. Un inconvénient de l'état de la technique est en effet que ces étapes sont réalisées de manière séquentielle. Un autre inconvénient est que la réaction d'interaction spécifique utilisée pour l'étape de confirmation, réaction immunologique ou réaction d'hybridation moléculaire, est, le plus souvent, lue en « point final ». Pendant ce temps, dans l'industrie agroalimentaire, la totalité du lot de produit fini est bloquée en attendant le résultat de la confirmation, et en clinique, la mise en place de l'antibiothérapie pertinente et des mesures de prévention est retardée.

Au vu de l'état de la technique considéré, il manque donc un procédé combinant les étapes d'enrichissement, de détection et d'identification précise. Concrètement, un tel procédé conjuguerait rapidité, spécificité, et sensibilité.

La présente invention propose donc de pallier les inconvénients décrits *supra* en mettant en oeuvre simultanément une culture des microorganismes et au moins une réaction d'agglutination, en milieu liquide.

De façon plus précise, l'invention concerne en premier lieu un procédé de détection et d'identification d'au moins un microorganisme susceptible d'être présent dans un échantillon, comprenant les étapes consistant à :
a) mettre en contact dans un conteneur : un milieu de culture permettant la croissance et/ou la détection des microorganismes, ledit échantillon et un support solide sensibilisé ;
b) soumettre l'ensemble à une température favorisant la croissance et/ou la détection des microorganismes ;
c) observer en temps réel l'apparition d'une agglutination indiquant la présence du ou des microorganismes ou confirmant ladite présence lorsque lesdits microorganismes sont détectés dans ledit milieu de culture, à l'issue de l'étape b).

Un autre objet de l'invention concerne un procédé de détection et d'identification d'au moins un microorganisme susceptible d'être présent dans un échantillon, comprenant les étapes consistant à :
a) mettre en contact dans un conteneur : un milieu de culture permettant la croissance et/ou l'identification des microorganismes, ledit échantillon et un support solide sensibilisé ;
b) soumettre l'ensemble à une température favorisant la croissance et/ou l'identification des microorganismes ; et
c) observer en temps réel l'apparition d'une agglutination permettant de réaliser l'identification du ou des microorganismes ou de confirmer ladite identification, lorsque le ou lesdits microorganismes sont identifiés dans ledit milieu de culture, à l'issue de l'étape b).

L'invention concerne par ailleurs, un procédé de détection et d'identification d'au moins un microorganisme susceptible d'être présent dans un échantillon, comprenant les étapes consistant à :
a) mettre en contact dans un conteneur : un milieu de culture permettant la croissance et l'identification des microorganismes, ledit échantillon et un support solide sensibilisé ;
b) soumettre l'ensemble à une température favorisant la croissance et l'identification des microorganismes ; et
d) observer en temps réel l'apparition d'une agglutination permettant de compléter l'identification du ou des microorganismes, réalisée à l'issue de l'étape b).

Par compléter l'identification, on entend apporter une information supplémentaire permettant de préciser l'identification du microorganisme. Par exemple, à l'étape a) , le milieu de culture utilisé peut être spécifique des bactéries du genre *Escherichia coli* et comporter un substrat spécifique de ce genre bactérien, de sorte que la présence de telles bactéries dans l'échantillon à tester, se caractérise par une modification du milieu de culture, tel un changement de couleur, si le substrat utilisé est un substrat chromogène. L'agglutination observée à l'étape c) peut, par exemple, permettre de mettre en évidence une souche particulière du genre *Escherichia coli,* telle *E. coli* 0157:H7, qui est une souche entéropathogène.

La température favorisant la croissance des microorganismes est comprise entre 20 et 44°C et l'échantillon est maintenu à cette température pendant une durée suffisante pour permettre la détection des microorganismes, soit une durée comprise entre 6 et 96 heures.

La mise en oeuvre combinée de ces différentes techniques et dans un conteneur unique permet à la fois de gagner du temps et de limiter les manipulations, donc les contaminations des manipulateurs ou des échantillons, entraînant dans ce dernier cas des faux-positifs. En outre, la mise en oeuvre de l'invention peut être automatisée. On notera également que le gain de temps est lié à la fois à la combinaison de deux étapes en une seule et à la détection de l'agglutination en temps réel et non plus en point final comme dans les techniques de confirmation mentionnées *supra.*

Avantageusement, les étapes a) et c) des procédés décrits *supra* mettent en oeuvre des composés chromogènes (également dénommés chromophores) ou fluorescents (également dénommés fluorophores).

Plus particulièrement, les deux procédés de détection et d'identification peuvent préférentiellement mettre en oeuvre l'apparition ou la disparition d'une coloration ou d'une fluorescence. Par ailleurs, dans tous les procédés objets de l'invention, l'agglutination peut avantageusement être mise en évidence par l'apparition ou la disparition d'une coloration ou d'une fluorescence.

De façon préférentielle, le conteneur est pris dans le groupe constitué par les microplaques, les microcupules, les microtubes, les capillaires ou les cartes multipuits.

Avantageusement, le procédé objet de l'invention peut en outre comporter une étape d'énumération des microorganismes, de préférence selon la méthode du nombre le plus probable explicitée dans le brevet EP 1 105 457 de la Demanderesse.

Selon un mode particulier de réalisation, la réaction d'agglutination mise en oeuvre à l'étape c) est une réaction d'immuno-agglutination, mettant en évidence une réaction antigène-anticorps.

Selon un autre mode particulier de réalisation, la réaction d'agglutination mise en oeuvre à l'étape c) est une réaction de type phage - bactérie. Plus particulièrement, il s'agit d'une réaction entre une protéine recombinante de phage spécifique d'un type bactérien et la molécule bactérienne correspondante. De telles interactions sont décrites dans le brevet EP 1 198 713.

Selon un autre mode particulier de réalisation, la réaction d'agglutination mise en oeuvre à l'étape c) est une réaction de type ligand/antiligand.

Selon un autre mode particulier de réalisation, la détection en temps réel de la réaction d'agglutination mise en oeuvre à l'étape c) selon l'un des modes de réalisation décrits *supra* peut permettre de détecter une sédimentation avant apparition de l'agglutination.

En outre, un kit de diagnostic permettant la mise en oeuvre du procédé suivant les différents modes de réalisation développés *supra* est également décrit. Le kit comporte :
- un conteneur ;
- un milieu de culture sélectif ou non, ledit milieu de culture contenant ou non un substrat spécifique du métabolisme du genre ou de l'espèce microbienne à détecter ; et
- un support solide sensibilisé.

De façon avantageuse, le conteneur est pris dans le groupe constitué par les microplaques, les microcupules, les microtubes, les capillaires ou les cartes multipuits.

Selon un premier mode de réalisation préférentiel, le support sensibilisé est un complexe support solide-antigène ou support solide anticorps.

Selon un deuxième mode de réalisation préférentiel, ledit support sensibilisé est un complexe support solide - ligand ou support solide - antiligand. Le ligand peut comporter tout ou partie d'un bactériophage.

Le kit de diagnostic selon l'invention peut comporter en outre au moins un composé chromogène ou fluorescent.

Enfin, un dernier objet de l'invention concerne l'utilisation d'un kit de diagnostic pour détecter et/ou identifier au moins un microorganisme susceptible d'être présent dans un échantillon est également décrit.

L'invention sera mieux comprise à la lecture de la description détaillée et des exemples non limitatifs qui suivent, en combinaison avec les dessins dans lesquels :
- La figure 1 représente une galerie de test pour la caractérisation de l'origine bactérienne d'une mammite/mastite, avant incubation.
- La figure 2 représente la galerie de test après incubation, avec une réaction positive à *Staphylococcus aureus.*
- La figure 3 représente la galerie de test après incubation, avec une réaction positive à *Streptococcus spp.*
- La figure 4 représente la galerie de test après incubation, avec une réaction positive à *Escherichia coli.*
- La figure 5 représente la galerie de test après incubation, avec une réaction positive à *Klebsiella spp.*

Le procédé objet de l'invention peut être utilisé pour des échantillons d'origine alimentaire, environnementale ou clinique. L'échantillon est défini comme une petite partie ou petite quantité isolée d'une entité pour l'analyse.

Parmi les échantillons d'origine alimentaire, on peut citer de façon non exhaustive un échantillon de produits lactés (yaourts, fromages...), de viande, de poisson, d'oeufs, de fruits, de légumes, d'eau, de boisson (lait, jus de fruits, soda, etc). Ces échantillons d'origine alimentaire peuvent aussi provenir de sauces ou de plats élaborés. Un échantillon alimentaire peut enfin être issu d'une alimentation destinée aux animaux, telle que notamment des farines animales.

On mentionnera aussi les échantillons liés à l'environnement tels les prélèvements de surface, d'eau, d'air.

Les échantillons d'origine clinique peuvent correspondre à des prélèvements de fluides biologiques (sang total, sérum, plasma, urine, liquide céphalo-rachidien), de selles, des prélèvements de nez, de gorge, de peau, de plaie, d'organes, de tissus ou de cellules isolées etc.

Le contrôle microbiologique correspond à l'analyse d'un échantillon dans le but d'isoler et/ou d'identifier et/ou d'énumérer des microorganismes potentiellement présents tels des bactéries ou des levures. Techniquement, cette analyse comprend la croissance *in vitro* des microorganismes dans un milieu de culture. Par milieu de culture, on entend un milieu comprenant tous les éléments nécessaires à la survie et/ou à la croissance des microorganismes. Le milieu de culture peut contenir d'éventuels additifs comme par exemple : des peptones, un ou plusieurs facteurs de croissance, des hydrates de carbone, un ou plusieurs agents sélectifs, des tampons, un ou plusieurs gélifiants etc. Ce milieu de culture peut se présenter sous forme de liquide, de gel prêt à l'emploi, c'est-à-dire prêt à l'ensemencement en tube, flacon ou sur boite de Pétri.

Au sens de la présente invention, le terme microorganisme recouvre les bactéries gram positif ou gram négatif, les levures et plus généralement, les organismes unicellulaires, invisibles à l'oeil nu, qui peuvent être manipulés et multipliés en laboratoire.

D'une manière générale, le milieu de culture peut en sus contenir un substrat permettant la détection d'une activité enzymatique ou métabolique des microorganismes cibles grâce à un signal détectable directement ou indirectement. Pour une détection directe, ce substrat peut être lié à une partie faisant office de marqueur, fluorescent ou chromogène. Pour une détection indirecte, le milieu de culture selon l'invention peut comporter en sus un indicateur de pH, sensible à la variation de pH induite par la consommation du substrat et révélant la croissance des microorganismes cibles. Ledit indicateur de pH peut être un chromophore ou un fluorophore. On citera comme exemples de chromophores le rouge neutre, le bleu d'aniline, le bleu de bromocresol. Les fluorophores comprennent par exemple la 4-méthylumbelliferone, les dérivés de l'aminocoumarine ou les dérivés de la résorufine.

Au sens de la présente invention, l'identification du microorganisme recherché, potentiellement effectuée par la recherche de ses caractères métaboliques, doit être confirmée. Cette confirmation peut faire appel à des réactions d'agglutination.

Par agglutination, on entend le résultat d'une interaction entre des microorganismes et des particules, lesdites particules étant soit d'origine naturelle telles des immunoglobulines de type M, soit de type support solide telles des polymères. Par cette interaction, microorganismes et particules s'agrègent, adhèrent entre eux et forment un réseau. Ledit réseau est susceptible de sédimenter ou de précipiter. L'interaction entre les microorganismes et les particules peut entraîner une sédimentation préalable, que l'observation en temps réel permettra de détecter avant la formation complète du réseau. Les réactions d'agglutination comprennent des réactions immunologiques, telles les réactions antigène-anticorps ou plus généralement des interactions spécifiques entre des protéines. Le réseau ou complexe formé par ladite réaction spécifique est alors détecté soit visuellement, soit de façon automatique grâce à un système optique. Alternativement, la quantité de complexe formée peut être déterminée.

L'une quelconque des différentes méthodes connues de l'homme de l'art pour effectuer une réaction d'agglutination peut être mise en oeuvre. Le support solide est choisi parmi les matériaux naturels, les matériaux de synthèse modifiés ou non chimiquement, et notamment parmi les latex, les polymères de type polychlorure de vinyle, polyéthylène, polystyrène, polyacrylate et les copolymères du type de ceux à base de styrène. Un tel support solide peut être sous forme de particules.

Par « support sensibilisé », on entend la fixation sur ledit support solide de composés fonctionnels comprenant des antigènes, des anticorps, des phages entiers ou des protéines de phages.

Le terme antigène désigne un composé susceptible d'être reconnu par un anticorps dont il a induit la synthèse par une réponse immune.

Le terme anticorps inclut les anticorps polyclonaux ou monoclonaux, les anticorps obtenus par recombinaison génétique et des fragments d'anticorps.

Les phages, ou bactériophages, sont des virus n'infectant que des bactéries ; ils sont également appelés virus bactériens. Dans les réactions d'agglutinations, ils sont utilisés pour leurs protéines qui reconnaissent une souche ou une espèce bactérienne donnée avec une grande spécificité et une grande sensibilité.

La fixation sur un support solide peut correspondre à une immobilisation directe ou indirecte : par immobilisation directe, on entend la fixation par covalence ou adsorption passive ; une immobilisation directe peut être effectuée au moyen d'un ligand fixé chimiquement sur ledit support solide ; par immobilisation indirecte, on entend l'interaction ligand/antiligand entre un ligand fixé sur l'antigène, l'anticorps ou le phage (plus largement, le composé fonctionnel) et l'antiligand ou ligand complémentaire fixé sur le support solide.

Les couples ligand/antiligand sont bien connus de l'homme du métier, et on peut citer par exemple les couples suivants : biotine/streptavidine, haptène/anticorps, antigène/anticorps, peptide/anticorps, sucre/lectine, polynucléotide/complémentaire du polynucléotide. Un composé hydrosoluble dérivé d'un homopolymère ou copolymère d'anhydride maléique tels ceux développés par la demanderesse dans le brevet délivré EP 0 561 722 peut également être utilisé pour immobiliser une molécule biologique. Ces supports solides peuvent être distribués dans la réaction sous différentes formes : lyophilisée, en suspension liquide, sous forme de bille telle que celles disponibles commercialement sous la marque BioBall® etc. L'agglutination peut être détectée visuellement, ou par un lecteur optique automatique selon différents principes connus de l'homme du métier parmi lesquels on citera, de manière non exhaustive :
(1) la détection de l'apparition d'une fluorescence, par sédimentation d'un latex coloré absorbant la fluorescence initialement présente dans le milieu ;
(2) la détection d'un changement de couleur par mélange des particules de latex colorées avec la matrice potentiellement colorée ;
(3) la concentration de fluorescence par agglutination des particules de latex fluorescentes, entraînant une disparition de la fluorescence diffuse dans le milieu ;
(4) la disparition de couleur par sédimentation d'un latex coloré présent dans le milieu.

Les fluorophores utilisables ont été mentionnés *supra* et comprennent la 4-méthylumbelliferone, les dérivés de l'aminocoumarine ou les dérivés de la résorufine.

Les opérations de culture/identification puis agglutination en point final décrites en deux étapes sont, selon la présente invention, combinées en une seule étape, l'agglutination spécifique étant détectée en temps réel. A titre d'illustration, on citera l'identification possible dans un échantillon d'origine alimentaire de *Listeria monocytogenes,* serotype 4b : la détection de *Listeria monocytogenes* peut être effectuée à l'aide d'un milieu de culture contenant un ou des substrats spécifiques du métabolisme de cette bactérie et l'identification du sérotype 4b sera effectuée simultanément par la réaction d'agglutination, détectée en temps réel.

Dans un mode de réalisation particulier, l'invention peut être mise en oeuvre dans des conteneurs tels des microplaques, des microcupules, des microtubes, des capillaires etc.

Avantageusement, le procédé selon l'invention peut être associé à un dispositif automatique de contrôle microbiologique de type TEMPO@ tel que développé par la demanderesse et peut facultativement permettre une énumération des microorganismes détectés.

Le procédé, objet de l'invention, peut être mis en oeuvre à l'aide d'un kit comprenant : un milieu réactionnel contenant une base nutritive, des particules sensibilisées et éventuellement un substrat chromogène spécifique du ou des microorganismes recherchés. Ledit milieu est remis en suspension par un aliquot de l'échantillon à analyser. Avantageusement, le kit permettant de mettre en oeuvre le procédé selon l'invention peut aussi contenir un conteneur solide de type microplaque, microtube, microcupule, capillaire, carte VITEK® ou carte TEMPO@.

### EXEMPLES

### Exemple 1 : Double numération Escherichia coli spp et Escherichia coli 0157 en associant réactions phénotypiques et immunologiques

Le but de cette analyse est de faire la numération simultanée de *E. coli spp* par voie enzymatique et de *E.coli O157* par voie immunologique en utilisant le système TEMPO@ commercialisé par la demanderesse

### Mode opératoire :

### Etape 1 : remise en suspension du milieu réactionnel par un aliquot de l'échantillon à analyser :

Le milieu réactionnel contient :
- Un substrat enzymatique spécifique de l'espèce *E.coli :* 4-méthylumbelliféryl-β-D-glucuronide (Biosynth réf. M-5700) : non fluorescent à T0) à 50 mg/l (peut varier entre 0,1 mg/l et 1000 mg/1); ;
- Des particules de latex (Oxoïd, réf. DR0620M), colorées en bleu, sensibilisées avec des anticorps spécifiques de *E. coli* 0157 à 1% d'extrait sec (peut varier entre 0.1 et 10% );
- Une base nutritive : peptones à la concentration de 10 g/l ;
- Un système inhibiteur : sels biliaires à la concentration de 1,5 g/l

Le milieu réactionnel resuspendu dans l'échantillon (e.g 4ml d'eau du réseau de distribution) est ensuite incorporé dans une carte TEMPO@ en vue de réaliser une numération.

Avant incubation de la carte, les puits de cette dernière sont bleus et non-fluorescents.

### Etape 2 : incubation de la carte :

Les cartes TEMPO@ sont ensuite incubées à 37°C pendant 24h. Au cours de cette période d'incubation, la réaction d'agglutination des particules sensibilisées avec des anticorps anti- *E.coli O157* et la réaction enzymatique (dégradation du substrat spécifique d*'E.coli*) s'effectuent simultanément à la croissance bactérienne, en cas de présence des bactéries cibles dans les puits de la carte TEMPO@.

### Etape 3 : lecture du test après 24h d'incubation :

Réaction positive à *E. coli O157,* il y a formation du complexe Anticorps anti-*E*. *coli* 0157-cellules d*'E.coli O157* se traduisant par l'agglutination des particules de latex et par conséquent une disparition de la coloration bleue, consécutive à la formation d'un précipité bleu au fond des puits concernés.

Réaction positive à *E.coli spp :* apparition de fluorescence (libération de molécules fluorescentes de 4-méthylumbelliférone) dans le/les puits positifs après dégradation du substrat par *E.coli spp.*

Puits positifs à *E.coli spp* et négatif à *E.coli O157:* Fluorescents et bleus
Puits positifs à *E.coli spp* et *E.coli O157 :* Fluorescents et non colorés (+ précipité bleu au fond du puits)
Puit négatif à *E.coli spp* et *E.coli O157:* Non fluorescents et bleus

Les puits positifs *E.coli O157* et *E.coli spp* sont ensuite comptabilisés pour déterminer *via* la table NPP (algorithme interne) le nombre d'unités formant colonies (UFC) de *E.coli O157* et *E.coli spp* par gramme d'échantillon.

### Exemple 2 : Caractérisation de l'origine bactérienne d'une mammite/mastite chez les bovins/ovins/caprins laitiers en vue d'adopter un traitement antibiotique approprié et efficace

La mammite résulte d'une infection de la mamelle par des bactéries plus ou moins adaptées à ce biotope. Plusieurs bactéries sont responsables de ce type infection et sont classées en deux groupes : les bactéries à réservoirs mammaires (e.g *Staphylococcus aureus, Streptococcus spp*) et les bactéries de l'environnement (e.g *Escherichia coli, Klebsiella spp).*

Principe du test : caractérisation de l'origine bactérienne d'une mammite par agglutination de particules sensibilisées, en suspension dans un milieu liquide, avec les cellules bactériennes cibles (i.e *Staphylococcus aureus, Streptococcus spp, Escherichia coli, Klebsiella spp).*

### Mode opératoire :

### Etape 1 : remisse en suspension du milieu réactionnel par un aliquot de l'échantillon à analyser (e.g lait) :

Le test se présente sous la forme d'une galerie comportant 4 puits contenant le milieu réactionnel.

La composition du milieu réactionnel est la suivante :
- Des particules bleues sensibilisées avec des protéines de phages et/ou des anticorps (Ac) spécifiques des cibles : *Staphylococcus aureus* (puits N°1) , *Streptococcus spp* (puits N°2), *Escherichia coli* (puits N°3), *Klebsiella spp* (puits N°4) ;
- Une base nutritive : Eau Peptonnée Tamponnée (Ref bMx 51094)

Chaque puit est resuspendu avec 500µl d'échantillon.

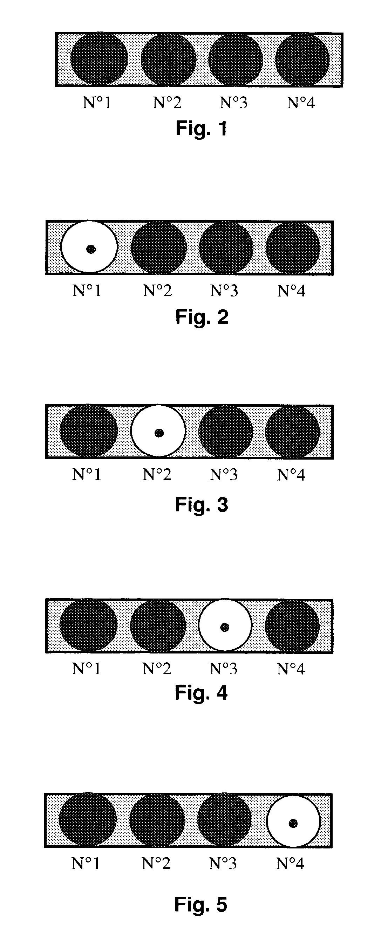

Etat initial de la galerie avant incubation (absence de réaction) : tous les puits de la galerie sont bleus étant donné l'absence d'agglutination et de sédimentation à T0 des particules bleues sensibilisées anti-cible (cf figure 1).

### Etape 2 : incubation, de la galerie:

La galerie est ensuite incubée à 37°C pendant 4 à 16h. Au cours de cette période d'incubation, la réaction d'agglutination des particules sensibilisées anti-cibles s'effectue simultanément à la croissance bactérienne, en cas de contamination du puits par la bactérie cible responsable de la mammite investiguée.

### Etape 3 : lecture du test après 4 à 16h d'incubation:

Réaction positive : formation du complexe protéine de phage/bactérie ou Anticorps/ antigène bactérien se traduisant par l'agglutination des particules de latex (formation d'un précipité au fond du puits) et par conséquent une disparition de la coloration bleue dans les puits concernés. Différents cas sont envisageables :
- Dans le cas où la mammite est due à *Staphylococcus aureus,* la disparition de la coloration bleue se produit dans le puit N°1. (cf. figure 2).
- Dans le cas où la mammite est due à *Streptococcus spp,* la disparition de la coloration bleue se produit dans le puit N°2 (cf. figure 3).
- Dans le cas où la mammite est due à *Escherichia coli,* la disparition de la coloration bleue se produit dans le puit N°3 (cf. figure 4).
- Dans le cas où la mammite est due à *Klebsiella spp,* la disparition de la coloration bleue se produit dans le puit N°4 (cf. figure 5).

### Exemple 3 : numération de Salmonella sur la plateforme TEMPO®

Le but de cette analyse est de dénombrer les *Salmonella* par voie immunologique par agglutination de particules sensibilisées avec des protéines recombinantes de phages, en utilisant le système TEMPO@ commercialisé par la demanderesse.

### Mode opératoire :

### Etape 1 : adsorption des protéines de phage anti salmonella B D1

Cette étape consiste à adsorber les protéines de phage à la surface de particules :
- référence: protéines de phage **anti B-D1** 2,67 mg/ml
- Latex utilisé, fourni par Polymer Laboratories : Plain fluorescent yellow PL FY lot CD 222 :311nm

Les protéines de phage sont adsorbées à la concentration de 140 µg/ml en tampon phosphate 20 mM pH 7.2. Le volume de l'essai est de 200 µl. Les particules de latex sont introduites à raison de 5 mg/ml dans le milieu.

L'adsorption est réalisée sous agitation sur une roue tournante durant 15 heures. Contrôle du rendement d'adsorption :
- centrifugation de 50 µl d'échantillon et récupération du surnageant ;
- dosage du surnageant par la méthode BCA de dosage des protéines ;
- rendement calculé par rapport à la courbe étalon et par rapport à la concentration initiale de la protéine de phage utilisée pour l'essai.

Ce rendement est de 73%.

La taille des particules est mesurée au granulomètre.

Elle est de 311 nm pour le latex PL FY et de 315 nm pour les conjugués particules-protéines de phage.

### Etape 2 : incubation dans la carte TEMPO® :

Le milieu réactionnel contient :
- 3.5 ml de base nutritive : peptones à la concentration de 10 g/l
- un échantillon contenant 50 UFC de bactéries *Salmonelle bareilly* 06.03.927 IM1272 : **C₁**
- 0.5 ml de particules de latex fluorescentes, sensibilisées avec des protéines de phage spécifiques de *Salmonella* à 0.5% d'extrait sec ;

Le milieu réactionnel est ensuite incorporé dans une carte TEMPO@ en vue de réaliser une numération.

Avant incubation de la carte, les puits de cette dernière sont fluorescents.

Les cartes TEMPO@ sont ensuite incubées à 37°C pendant 24h. Au cours de cette période d'incubation, la réaction d'agglutination des particules sensibilisées avec les protéines de phage anti-B-D1 s'effectue simultanément à la croissance bactérienne, en cas de présence des bactéries cibles dans les puits de la carte TEMPO®.

### Etape 3 : lecture du test après 24h d'incubation:

Réaction positive à *Salmonella bareilly,* il y a formation du complexe protéines de phage-cellules de *Salmonella bareilly* se traduisant par l'agglutination et la sédimentation des particules de latex. En conséquence, on observe une concentration de la fluorescence au fond des puits concernés et une disparition de la fluorescence au niveau de la fenêtre de lecture.

Réaction négative : la fluorescence reste homogène dans tout le puits.

Les puits positifs sont ensuite comptabilisés pour déterminer *via* la table NPP (algorithme interne) le nombre d'unités formant colonies (UFC) de *Salmonella bareilly* 06.03.927 IM1272: **C₁** par gramme d'échantillon.

Le résultat de dénombrement est de 11 UFC/ml soit 44 UFC introduites, en accord avec les 50 UFC théoriques.

## Revendications

1. Procédé de détection dans un conteneur d'au moins un microorganisme susceptible d'être présent dans un échantillon, comprenant les étapes consistant à :
a) mettre en contact dans ledit conteneur : un milieu de culture permettant la croissance et la détection des microorganismes, ledit échantillon et un support solide sensibilisé ;
b) soumettre l'ensemble à une température favorisant la croissance et la détection des microorganismes ; et
c) observer en temps réel, à l'intérieur dudit conteneur et simultanément à la croissance du ou des microorganismes, l'apparition d'une agglutination indiquant la présence du ou des microorganismes ou confirmant ladite présence lorsque lesdits microorganismes sont détectés dans ledit milieu de culture, à l'issue de l'étape b).

2. Procédé de détection et d'identification dans un conteneur d'au moins un microorganisme susceptible d'être présent dans un échantillon, comprenant les étapes consistant à :
a) mettre en contact dans ledit conteneur : un milieu de culture permettant la croissance et l'identification des microorganismes, ledit échantillon et un support solide sensibilisé ;
b) soumettre l'ensemble à une température favorisant la croissance et l'identification des microorganismes ; et
c) observer en temps réel à l'intérieur dudit conteneur et simultanément à la croissance du ou des microorganismes, l'apparition d'une agglutination permettant de réaliser l'identification du ou des microorganismes ou de confirmer ladite identification, lorsque le ou lesdits microorganismes sont identifiés dans ledit milieu de culture, à l'issue de l'étape b).

3. Procédé de détection et d'identification dans un conteneur unique d'au moins un microorganisme susceptible d'être présent dans un échantillon, comprenant les étapes consistant à :
a) mettre en contact dans ledit conteneur : un milieu de culture permettant la croissance et l'identification des microorganismes, ledit échantillon et un support solide sensibilisé ;
b) soumettre l'ensemble à une température favorisant la croissance et l'identification des microorganismes ; et
c) observer en temps réel à l'intérieur dudit conteneur et simultanément à la croissance du ou des microorganismes, l'apparition d'une agglutination permettant de compléter l'identification du ou des microorganismes, réalisée à l'issue de l'étape b).

4. Procédé selon l'une des revendications précédentes, dans lequel la détection ou l'identification dans le milieu de culture met en oeuvre l'apparition ou la disparition d'une coloration ou d'une fluorescence.

5. Procédé selon l'une des revendications précédentes, dans lequel l'agglutination est mise en évidence par l'apparition ou la disparition d'une coloration ou d'une fluorescence.

6. Procédé selon l'une des revendications précédentes, dans lequel le conteneur est pris dans le groupe constitué par les microplaques, les microcupules, les microtubes, les capillaires ou les cartes multipuits.

7. Procédé selon l'une des revendications précédentes, comportant en outre une étape d'énumération des microorganismes.

8. Procédé selon l'une des revendications précédentes, dans lequel la réaction d'agglutination met en oeuvre une réaction antigène-anticorps.

9. Procédé selon l'une des revendications 1 à 7, dans lequel la réaction d'agglutination met en oeuvre une réaction de type phage-protéine bactérienne.

10. Procédé selon l'une des revendications 1 à 7 dans lequel la réaction d'agglutination met en oeuvre une réaction de type ligand/antiligand.

## Patentansprüche

1. Verfahren zum Nachweisen von mindestens einem Mikroorganismus, von dem man vermutet, dass er in einer Probe vorhanden ist, in einem Behältnis, umfassend die Schritte, die aus Folgendem bestehen:
a) Inkontaktbringen in dem Behältnis von einem Kulturmedium, das das Wachstum und den Nachweis von Mikroorganismen gestattet, der Probe sowie einem festen sensibilisierten Träger;
b) Aussetzen des Ganzen gegenüber einer Temperatur, die das Wachstum und den Nachweis von Mikroorganismen begünstigt; und
c) Beobachten in Echtzeit das Auftreten einer Agglutinierung im Inneren des Behältnisses und gleichzeitig mit dem Wachstum des bzw. der Mikroorganismen, die das Vorhandensein von dem bzw. den Mikroorganismen anzeigt oder das Vorhandensein bestätigt, wenn die Mikroorganismen in dem Kulturmedium am Ende von Schritt b) nachgewiesen werden.

2. Verfahren zum Nachweisen und zum Identifiieren von mindestens einem Mikroorganismus, von dem man vermutet, dass er in einer Probe vorhanden ist, in einem Behältnis, umfassend die Schritte, die aus Folgendem bestehen:
a) Inkontaktbringen in dem Behältnis von einem Kulturmedium, das das Wachstum und die Identifikation von Mikroorganismen gestattet, der Probe sowie einem festen sensibilisierten Träger;
b) Aussetzen des Ganzen gegenüber einer Temperatur, die das Wachstum und die Identifikation von Mikroorganismen begünstigt; und
c) Beobachten in Echtzeit das Auftreten einer Agglutinierung im Inneren des Behältnisses und gleichzeitig mit dem Wachstum des bzw. der Mikroorganismen, die das Identifizieren des bzw. der Mikroorganismen oder das Bestätigen der Identifikation gestattet, wenn der bzw. die Mikroorganismen in dem Kulturmedium am Ende von Schritt b) identifiziert worden sind.

3. Verfahren zum Nachweisen und zum Identifiieren von mindestens einem Mikroorganismus, von dem man vermutet, dass er in einer Probe vorhanden ist, in einem einzigen Behältnis, umfassend die Schritte, die aus Folgendem bestehen:
a) Inkontaktbringen in dem Behältnis von einem Kulturmedium, das das Wachstum und die Identifikation von Mikroorganismen gestattet, der Probe sowie einem festen sensibilisierten Träger;
b) Aussetzen des Ganzen gegenüber einer Temperatur, die das Wachstum und die Identifikation von Mikroorganismen begünstigt; und
c) Beobachten in Echtzeit das Auftreten einer Agglutinierung im Inneren des Behältnisses und gleichzeitig mit dem Wachstum des bzw. der Mikroorganismen, die es ermöglicht, die Identifizierung des bzw. der Mikroorganismen, die am Ende von Schritt b) erfolgt worden ist, zu vervollständigen.

4. Verfahren nach einem der vorhergehenden Ansprüche, bei dem man sich für den Nachweis oder die Identifikation in dem Kulturmedium des Auftretens oder Verschwindens einer Färbung oder einer Fluoreszenz bedient.

5. Verfahren nach einem der vorhergehenden Ansprüche, bei dem die Agglutination durch das Auftreten oder Verschwinden einer Färbung oder einer Fluoreszenz nachgewiesen wird.

6. Verfahren nach einem der vorhergehenden Ansprüche, bei dem das Behältnis aus der Gruppe bestehend aus Mikroplatten, Mikronäpfchen, Mikroröhrchen, Kapillarröhrchen oder Multiwell-Platten ausgewählt ist.

7. Verfahren nach einem der vorhergehenden Ansprüche, weiterhin umfassend einen Schritt des Aufzählens der Mikroorganismen.

8. Verfahren nach einem der vorhergehenden Ansprüche, bei dem bei der Agglutinierungsreaktion eine Antigen-Antikörper-Reaktion eingesetzt wird.

9. Verfahren nach einem der Ansprüche 1 bis 7, bei dem bei der Agglutinierungsreaktion eine Reaktion des Phagen-Bakterienprotein-Typs eingesetzt wird.

10. Verfahren nach einem der Ansprüche 1 bis 7, bei dem bei der Agglutinierungsreaktion eine Reaktion des Liganden-Antiliganden-Typs eingesetzt wird.

## Claims

1. Method for detecting in a container at least one microorganism that may be present in a sample, comprising the steps of:
a) bringing into contact, in said container: a culture medium that enables the growth and detection of microorganisms, said sample and a sensitized solid support;
b) subjecting the whole to a temperature that promotes the growth and detection of microorganisms; and
c) observing, in real time, inside said container, and simultaneously with the growth of the microorganism(s), the appearance of an agglutination indicating the presence of the microorganism(s) or confirming said presence when said microorganisms are detected in said culture medium, when step b) has been completed.

2. Method for detecting and identifying in a container at least one microorganism that may be present in a sample, comprising the steps of:
a) bringing into contact, in said container: a culture medium that enables the growth and identification of microorganisms, said sample and a sensitized solid support;
b) subjecting the whole to a temperature that promotes the growth and identification of microorganisms; and
c) observing, in real time, inside said container, and simultaneously with the growth of the microorganism(s), the appearance of an agglutination making it possible to identify the microorganism(s) or to confirm said identification, when said microorganism(s) is (are) identified in said culture medium, when step b) has been completed.

3. Method for detecting and identifying, in a single container, at least one microorganism that may be present in a sample, comprising the steps of:
a) bringing into contact, in said container: a culture medium that enables the growth and identification of microorganisms, said sample and a sensitized solid support;
b) subjecting the whole to a temperature that promotes the growth and identification of microorganisms; and
c) observing, in real time, inside said container, and simultaneously with the growth of the microorganism(s), the appearance of an agglutination making it possible to supplement the identification of the microorganism(s), made when step b) has been completed.

4. Method according to one of the preceding claims, in which the detection or the identification in the culture medium uses the appearance or the disappearance of a coloration or of a fluorescence.

5. Method according to one of the preceding claims, in which the agglutination is demonstrated by the appearance or the disappearance of a coloration or of a fluorescence.

6. Method according to one of the preceding claims, in which the container is taken from the group constituted of microplates, microcupules, microtubes, capillaries or multiwell cards.

7. Method according to one of the preceding claims, also comprising a step of counting the microorganisms.

8. Method according to one of the preceding claims, in which the agglutination reaction implements an antigen-antibody reaction.

9. Method according to one of Claims 1 to 7, in which the agglutination reaction implements a phage-bacterial protein reaction.

10. Method according to one of Claims 1 to 7, in which the agglutination reaction implements a ligand/antiligand reaction.
